(19) Europäisches Patentamt
European Patent Office
Office européen des brevets

(11) EP 1 521 623 B1

(12) **EUROPÄISCHE PATENTSCHRIFT**

(45) Veröffentlichungstag und Bekanntmachung des Hinweises auf die Patenterteilung:
**24.11.2010 Patentblatt 2010/47**

(21) Anmeldenummer: **03762574.6**

(22) Anmeldetag: **02.07.2003**

(51) Int Cl.:
**B01D 3/22** [(2006.01)]

(86) Internationale Anmeldenummer:
**PCT/EP2003/007026**

(87) Internationale Veröffentlichungsnummer:
**WO 2004/004861 (15.01.2004 Gazette 2004/03)**

(54) **VERFAHREN DER REKTIFIKATIVEN AUFTRENNUNG VON (METH) ACRYLMONOMERE ENTHALTENDEN FLUIDEN**

METHOD FOR THE RECTIFYING SEPARATION OF FLUIDS CONTAINING (METH)ACRYL MONOMERS

PROCEDE DE SEPARATION DE FLUIDES CONTENANT DES MONOMERES (METH)ACRYLIQUES PAR RECTIFICATION

(84) Benannte Vertragsstaaten:
**AT BE BG CH CY CZ DE DK EE ES FI FR GB GR HU IE IT LI LU MC NL PT RO SE SI SK TR**

(30) Priorität: **04.07.2002 DE 10230219**

(43) Veröffentlichungstag der Anmeldung:
**13.04.2005 Patentblatt 2005/15**

(73) Patentinhaber: **BASF SE**
**67056 Ludwigshafen (DE)**

(72) Erfinder:
• **HAMMON, Ulrich**
**68163 Mannheim (DE)**
• **THIEL, Joachim**
**67435 Neustadt (DE)**
• **SCHLIEPHAKE, Volker**
**67105 Schifferstadt (DE)**
• **SCHRÖDER, Jürgen**
**67071 Ludwigshafen (DE)**

(56) Entgegenhaltungen:
**EP-A- 1 029 573    EP-A- 1 279 429**
**US-A- 3 988 213**

• **HOPPE UND MITTELSTRASS: "Grundlagen der Dimensionierung von Kolonnenböden" 1967, VERLAG THEODOR STEINKOPF , DRESDEN * Seite 196 - Seite 211 ***
• **YUNG C.SHIN: "Handbook of Design, Manufacturing and Automation" 1994, JOHN WILEY & SONS INC. * Seite 243 - Seite 258 ***

**Beschreibung**

Vorliegende Erfindung betrifft ein Verfahren der rektifikativen Auftrennung von (Meth)acrylmonomere enthaltenden Fluiden (= das der Rektifikationskolonne zugeführte Fluid) in einer Rektifikationskolonne, die wenigstens einen Siebboden ohne Ablaufsegment enthält.

Die Schreibweise (Meth)acrylmonomere steht in dieser Schrift verkürzend für "Acrylmonomere und/oder Methacrylmonomere".

Der Begriff Acrylmonomere steht in dieser Schrift verkürzend für "Acrolein, Acrylsäure und/oder Ester der Acrylsäure".

Der Begriff Methacrylmonomere steht in dieser Schrift verkürzend für "Methacrolein, Methacrylsäure und/oder Ester der Methacrylsäure".

Im besonderen sollen die in dieser Schrift angesprochenen (Meth)acrylmonomeren die nachfolgenden (Meth)acrylsäureester umfassen: Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylacrylat und N,N-Dimethylaminoethylmethacrylat.

(Meth)acrylmonomere sind wichtige Ausgangsverbindungen zur Herstellung von Polymerisaten, die z.B. als Klebstoffe Verwendung finden.

(Meth)acrolein und (Meth)acrylsäure wird großtechnisch überwiegend durch katalytische Gasphasenoxidation geeigneter $C_3$-/$C_4$-Vorläuferverbindungen, insbesondere von Propen und Propan im Fall von Acrolein, Acrylsäure bzw. von iso-Buten und iso-Butan im Fall der Methacrylsäure und des Methacroleins, hergestellt. Neben Propen, Propan, iso-Buten und iso-Butan eignen sich als Ausgangsstoffe jedoch auch andere 3 bzw. 4 Kohlenstoffatome enthaltende Verbindungen, beispielsweise iso-Butanol, n-Propanol oder der Methylether (als $C_4$-Vorläufer) von iso-Butanol. (Meth)acrylsäure kann auch aus (Meth)acrolein erzeugt werden.

Dabei wird normalerweise ein Produktgasgemisch erhalten, aus dem die (Meth)acrylsäure bzw. das (Meth)acrolein abgetrennt werden muß.

Diese Abtrennung wird in der Regel so durchgeführt, daß die (Meth)acrylsäure bzw. das (Meth)acrolein durch Absorption in ein Lösungsmittel (z.B. Wasser oder ein organisches Lösungsmittel) oder durch fraktionierende Kondensation des Produktgasgemisches zunächst grundabgetrennt und das dabei anfallende Kondensat bzw. Absorbat nachfolgend rektifikativ (in der Regel in mehreren Stufen) unter Erhalt von mehr oder weniger reiner (Meth)acrylsäure bzw. (Meth)acrolein aufgetrennt wird (vgl. z.B. EP-A 717019, EP-A 1125912, EP-A 982289, EP-A 982287, DE-A 19606877, DE-A 1011527, DE-A 10224341, DE-A 10218419). Die vorstehend angesprochene fraktionierende Kondensation soll in dieser Schrift auch als unter den Begriff Rektifikation fallend betrachtet werden. Sie unterscheidet sich von der herkömmlichen Rektifikation lediglich dadurch, dass das aufzutrennende Gemisch der Trennkolonne (der Rektifikationskolonne) gasförmig (d.h. vollständig in die Dampfform überführt) zugeführt wird. Der in dieser Schrift verwendete Begriff Fluide soll deshalb sowohl Flüssigkeiten als auch Gasgemische umfassen.

Ester der (Meth)acrylsäure sind z.B. durch direkte Umsetzung von (Meth)acrylsäure und/oder (Meth)acrolein mit den entsprechenden Alkoholen erhältlich. Allerdings fallen auch in diesem Fall zunächst Produktgemische an, aus denen die (Meth)acrylsäureester z.B. rektifikativ abgetrennt werden müssen.

Die vorstehend angesprochenen, (Meth)acrylmonomere enthaltenden, Fluide bzw. Flüssigkeiten können die (Meth)acrylmonomere sowohl in mehr oder weniger reiner Form als auch in Lösung befindlich enthalten.

Das Lösungsmittel kann dabei sowohl wässrig als auch ein organisches Lösungsmittel sein. Die spezifische Art des Lösungsmittels ist erfindungsgemäß im wesentlichen unbeachtlich. Der Gehalt an (Meth)acrylmonomeren kann $\geq 2$, $\geq 5$ Gew.-%, oder $\geq 10$ Gew.-%, oder $\geq 20$ Gew.-%, oder $\geq 40$ Gew.-%, oder $\geq 60$ Gew.-%, oder $\geq 80$ Gew.-%, oder $\geq 90$ Gew.-%, oder $\geq 95$ Gew.-%, oder $\geq 99$ Gew.-% betragen.

Die rektifikative Auftrennung der beschriebenen (Meth)acrylmonomere enthaltenden Fluide bzw. Flüssigkeiten kann je nach ihrer Zusammensetzung sowohl so erfolgen, dass sich die (Meth)acrylmonomere am Kopf der Rektifikationskolonne anreichern, als auch so, dass sich die (Meth)acrylmonomere im Sumpf der Rektifikationskolonne anreichern. Selbstredend können auch im oberen, unteren oder mittleren Teil der Rektifikationskolonne die (Meth)acrylmonomere angereichert enthaltenden Fraktionen entnommen werden.

In allen Fällen (insbesondere den vorgenannten) können zur in Rede stehenden rektifikativen Auftrennung Rektifikationskolonnen verwendet werden, die wenigstens einen Siebboden ohne Ablaufsegment enthalten (vgl. z.B. DE-A 19924532). Selbstverständlich können diese Rektifikationskolonnen aber auch ausschließlich Siebböden ohne Ablaufsegment als alleinige trennwirksame Einbauten der Rektifikationskolonne enthalten (vgl. z.B. DE-A 10156988 und EP-A 1029573). Es können aber auch andere trennwirksame Einbauten wie z.B. Glockenböden oder Packungen mitverwendet werden.

Die Rektifikation kann dabei sowohl unter Normaldruck als auch unter reduziertem Druck durchgeführt werden. Typische Sumpftemperaturen liegen im Bereich von 100 bis 250°C und typische Kopfdrucke betragen 80 bis 500 mbar.

Die (Meth)acrylmonomere häufig begleitende Lösungsmittel sind vielfach Diphenyl enthaltend, z.B. Mischungen aus Diphenylether, Diphenyl und o-Dimethylphthalat. Ein z.B. für die Absorption von (Meth)acrylmonomeren häufig verwendetes Lösungsmittel enthält ca. 57,4 Gew.-% Diphenylether, 20,7 Gew.-% Diphenyl und 20 Gew.-% o-Dimethylphthalat.Andere (Meth)acrylsäure und (Meth)acrylsäureester häufig begleitende Lösungsmittel sind Methylacrylat und Ethylacrylat.

In der Regel wird bei den in dieser Schrift zur Rede stehenden Rektifikationen die Rektifikationskolonne mittels sogenannter Polymerisationsinhibitoren polymerisationsinhibiert. Diese werden üblicherweise am Kopf der Kolonne aufgegeben, können zusätzlich aber auch dem Sumpf zugefügt werden und auch bereits in der aufzutrennenden, die (Meth)acrylmonomere enthaltenden, Flüssigkeit zugesetzt sein. Typische Vertreter solcher Polymerisationsinhibitoren sind Phenothiazin, 2-Methoxyphenol und 4-Hydroxy-2,2,6,6-tetramethylpiperidin-N-oxyl. Bezogen auf den Gehalt der (Meth)acrylmonomeren werden häufig bis zu wenigen hundert Gew.-ppm an Polymerisationsinhibitoren eingesetzt.

Nachdem die für diese Schrift besonders relevanten (Meth)acrylmonomere enthaltenden Fluide bzw. Flüssigkeiten vorstehend näher beschrieben sind, soll näher auf die Siebböden ohne Ablaufsegment eingegangen werden. In der Literatur wird für solche Böden häufig auch der Begriff Dual-Flow-Böden verwendet. Darunter werden in dieser Schrift Platten mit einfachen Durchtrittsstellen (Löcher, Schlitze etc.) verstanden, die vielfach auch als Regensiebböden bezeichnet werden.

Durch die Abwesenheit von Ablaufsegmenten (Ablaufschächten) treten das aufsteigende Gas und die in der Kolonne absteigende Rücklaufflüssigkeit entgegengesetzt strömend durch die gleichen Durchtrittsstellen des Bodens. Der Querschnitt der Durchtrittsstellen wird in an sich bekannter Weise der Belastung der Kolonne angepasst. Ist er zu klein, strömt das aufsteigende Gas mit so hoher Geschwindigkeit durch die Durchtrittsstellen, dass die in der Kolonne absteigende Rücklaufflüssigkeit im wesentlichen ohne Trennwirkung mitgerissen wird. Ist der Querschnitt der Durchtrittsstellen zu groß, bewegen sich aufsteigendes Gas und absteigender Rücklauf im wesentlichen ohne Austausch aneinander vorbei und der Boden läuft Gefahr trocken zu laufen. D.h., der Arbeitsbereich für Dual-Flow-Böden ist durch 2 Grenzpunkte festgelegt. Eine minimale Grenzgeschwindigkeit muß vorhanden sein, damit auf dem Boden eine gewisse Flüssigkeitsschicht gehalten wird, um ein Arbeiten des Bodens zu ermöglichen. Die obere Grenze ist durch den Flutpunkt festgelegt, wenn die Geschwindigkeit zum Stau der Flüssigkeit auf den Böden führt und ein Durchregnen verhindert wird. Im normalen Arbeitsbereich regnet die in der Rektifikationskolonne rücklaufende Flüssigkeit in Tropfen von Dual-Flow-Boden zu Dual-Flow-Boden, d.h. zwischen den Böden wird die geschlossene Gasphase von einer zerteilten Flüssigkeitsphase durchsetzt. Die auf dem Regensiebboden auftreffenden Tropfen werden teilweise versprüht.

In der Regel schließt jeder Dual-Flow-Boden bündig mit den Kolonnenwänden ab. Es gibt aber auch Ausführungsvarianten, bei denen zwischen Kolonnenwand und Boden ein Zwischenraum besteht, der nur teilweise durch Brücken unterbrochen ist. Neben den eigentlichen Durchtrittsöffnungen weist er allenfalls noch Öffnungen auf, die z.B. eine Befestigung des Bodens auf Auflageringen oder ähnliches ermöglichen (Vgl. z.B. DE-A 10159823).

Die ältere Anmeldung DE-A 10156988 empfiehlt zur rektifikativen Behandlung von (Meth)acrylmonomere enthaltenden Flüssigkeiten Rektifikationskolonnen mit Dual-Flow-Böden mit Durchtrittsöffnungen, deren Querschnitt zwar innerhalb eines Dual-Flow-Bodens konstant ist, mit zunehmendem Abstand des Bodens vom Zulauf der zu behandelnden Flüssigkeit jedoch abnimmt.

Die Längsausdehnung der Durchtrittsöffnungen beträgt in typischer Weise 10 bis 80 mm gemäß der Lehre der DE-A 10156988. Nachteilig an der Lehre der DE-A 10156988 ist jedoch, dass sie keine Aussage darüber macht, wie die Durchtrittsöffnungen innerhalb eines Dual-Flow-Bodens relativ zueinander angeordnet werden sollen. Das gleiche gilt für die Lehre der EP-A 1029573. In beiden Schriften werden lediglich kreisförmige Bohrungen als bevorzugte Durchtrittsöffnungen empfohlen.

Intensive eigene Untersuchungen haben jedoch ergeben, daß die Relativanordnung der Durchtrittsöffnung eines Dual-Flow-Bodens von entscheidendem Einfluß auf die Trennleistung des Bodens bei einer rektifikativen Auftrennung von (Meth)acrylmonomere enthaltenden Fluiden bzw. Flüssigkeiten ist. Dies gilt insbesondere dann, wenn der Gehalt des Fluids bzw. der Flüssigkeit an (Meth)acrylatmonomeren 2 bis 5 Gew.-%, oder 10 bis 35 Gew.-%, oder $\geq$ 95 Gew.-% beträgt.

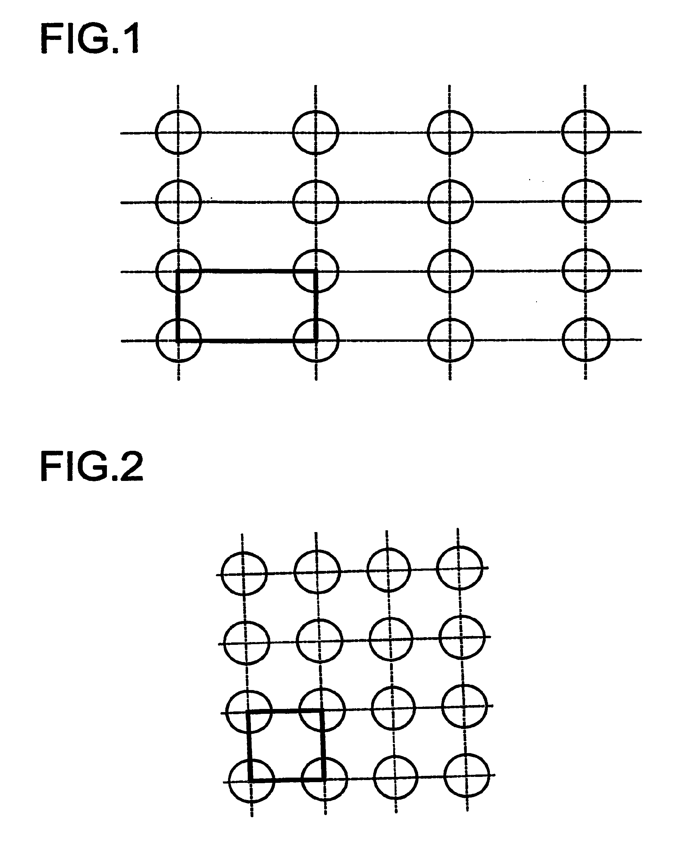

In diesem Zusammenhang wurde auch gefunden, daß die in Fig. 1 der EP-A 1029573 als mögliche Relativanordnung graphisch dargestellte unregelmäßige Dreiecksteilung (die Lage der Schwerpunkte der Bohrungen weicht von einer Lage auf gedachten Geraden deutlich ab) nicht voll zu befriedigen vermag.

Die Aufgabe der vorliegenden Erfindung bestand daher darin, für die rektifikative Auftrennung von (Meth)acrylmonomere enthaltenden Fluiden (insbesondere den im Vorstehenden dargestellten Fluiden) in einer Rektifikationskolonne, die wenigstens einen Siebboden ohne Ablaufsegment enthält, dahingehend eine verbesserte Relativanordnung der Durchtrittsöffnungen des Regensiebbodens zur Verfügung zu stellen, daß der Regensiebboden eine verbesserte Trennleistung aufweist.

Demgemäß wurde ein Verfahren der rektifikativen Auftrennung von (Meth)acrylmonomere enthaltenden Fluiden in einer Rektifikationskolonne, nach Anspruch 1 gefunden.

Zwei Kanten der Elementarzelle werden erfindungsgemäß dann als zueinander parallel betrachtet, wenn der von ihren Richtungen eingeschlossene Winkel ≤ 0,2°C beträgt. Bevorzugt beträgt der vorgenannte Winkel weniger als 10, besonders bevorzugt weniger als 5 und ganz besonders bevorzugt weniger als 2 Winkelminuten. Am besten beträgt dieser Einschlußwinkel 0°.

In ähnlicher Weise werden zwei parallele Kanten der Elementarzelle erfindungsgemäß dann als gleich lang betrachtet, wenn ihr Längenunterschied, bezogen auf die halbe Summe ihrer Längen, ≤ 1 %, bevorzugt ≤ 0,5 %, besonderes bevorzugt ≤ 0,25 %, und ganz besonders bevorzugt ≤ 0,1 % beträgt. Am besten sind die Längen identisch. Sind die Längen nicht gleich, ist die Länge des anspruchsgemäßen Verschiebungsvektors gleich der halben Längensumme. Im Normalfall wird die Elementarzelle nicht mehr als zehn Schwerpunkte von Durchtrittsöffnungen enthalten. Meist enthält sie ≤ 8, häufig ≤ 5 oder ≤ 4 Schwerpunkte von Durchtrittsöffnungen.

Im Normalfall ist diese Anzahl von Schwerpunkten einer Elementarzelle ≤ 10 %, meist ≤ 7 %, vielfach ≤ 5 % und häufig ≤ 3 % aller im Regensiebboden enthaltenen Schwerpunkte von Durchtrittsöffnungen.

Unter dem Schwerpunkt einer Durchtrittsöffnung soll der gedachte Massenschwerpunkt verstanden werden, der entsteht, wenn man die Durchtrittsöffnung mit einer homogenen Masse füllt, die an jeder Stelle der Öffnung die gleiche Fülldicke aufweist.

Prinzipiell können die Durchtrittsöffnungen des erfindungsgemäß mitzuverwendenden wenigstens eines Regensiebbodens jedwede geometrische Form aufweisen. D.h., die Durchtrittsöffnungen können Kreise, Ellipsen, Rechtecke, Dreiecke, Polygone oder Schlitze sein.

Erfindungsgemäß vorteilhaft weisen alle Durchtrittsöffnungen eines erfindungsgemäß mitzuverwendenden wenigstens einen Regensiebbodens die gleiche geometrische Form und den gleichen Querschnitt auf. In diesem Fall ist die Differenz zwischen dem größten und dem kleinsten Querschnitt der von der Elementarzelle erfassten Durchtrittsöffnungen bevorzugt ≤ 1 %, besser 5 0,75 %, noch besser 5 0,5 %, noch besser ≤ 0,25 %, noch besser ≤ 0,1 % und am besten 0 %, bezogen auf die Fläche des größten erfassten Querschnitts. Bevorzugt ist diese geometrische Form kreisförmig.

Selbstredend können sich innerhalb der erfindungsgemäß relevanten Elementarzellen aber auch Durchtrittsöffnungen von unterschiedlicher geometrischer Form und unterschiedlichem Querschnitt befinden.

Die erfindungsgemäß geforderte regelmäßige Anordnung der Schwerpunkte soll dann als gegeben betrachtet werden, wenn einerseits bei der regelmäßig wiederkehrenden Verschiebung der Elementarzelle entlang ihrer Kanten in wenigstens 90 % (bevorzugt wenigstens 95 %, besonders bevorzugt wenigstens 98 %, ganz besonders bevorzugt wenigstens 99 % bzw. 99,9 % und am besten 100 %) aller Fälle die Position des durch die Verschiebung erzeugten idealen Bildschwerpunktes und die Position des im Siebboden vorhandenen zugehörigen realen Schwerpunktes um ≤ 1 %, bevorzugt ≤ 0, 75 %, besonders bevorzugt um ≤ 0,5 %, ganz besonders bevorzugt um ≤ 0,3 % und noch besser um ≤ 0,1 % der Hälfte der Längen-Summe der beiden möglichen Verschiebungsvektoren voneinander entfernt sind (In der Regel wird dieser Abstand zwischen idealem und realem Schwerpunkt in der entsprechenden Anzahl der Fälle ≤ 0,1 mm, bevorzugt ≤ 0,05 mm und besonders bevorzugt 5 0, 02 mm betragen. Im Ideal fall fallen idealer und realer Schwerpunkt in allen Fällen zusammen.)und bei Erfüllung der jeweiligen vorstehenden Bedingung andererseits bei der regelmäßig wiederkehrenden Verschiebung der Elementarzelle entlang ihrer Kanten in wenigstens 90 % (bevorzugt wenigstens 95 %, besonders bevorzugt wenigstens 98 %, ganz besonders bevorzugt wenigstens 99 % bzw. 99,9 % und am besten 100 %) aller Fälle die Fläche der durch die Verschiebung erzeugten idealen Durchtrittsöffnung und die Fläche der im Siebboden vorhandenen zugehörigen realen Durchtrittsöffnung so weitgehend miteinander überlappen, daß die Summe aus der nicht überlappenden Restfläche der durch die Verschiebung erzeugten idealen Durchtrittsöffnung und der nicht überlappenden Restfläche der zugehörigen realen Durchtrittsöffnung, bezogen auf die Fläche der durch die Verschiebung erzeugten idealen Durchtrittsöffnung, (nachstehend $S_{Rest}$ genannt) ≤ 1 %, bevorzugt ≤ 0,75 %, besonders bevorzugt ≤ 0,5 %, ganz besonders bevorzugt ≤ 0,3 % und noch besser ≤ 0,1 % beträgt. Im Idealfall ist $S_{Rest}$ = O.

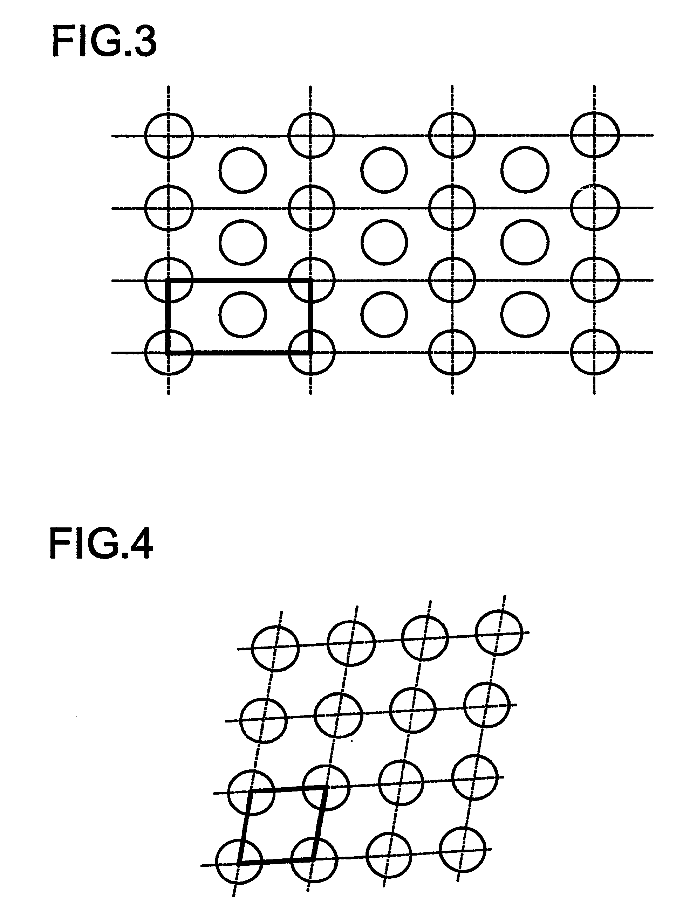

Erfindungsgemäß günstige Elementarzellen weisen die Geometrie eines Rechtecks (vgl. Fig. 1, 3 (rechteckig flächenzentrierte Elementarzelle) und 5), eines Quadrats (vgl. Fig. 2) oder einer Raute (vgl. Fig. 4) auf. Dies gilt auch dann, wenn in Fig. 3 zwischen der längeren Kante d und der kürzeren Kante a die Beziehung $d = a\sqrt{3}$ nicht erfüllt ist oder in Figur 4 die Kanten nicht gleich lang und/oder der eingeschloßene Winkel nicht 60° beträgt. Besonders bevorzugt ist eine Anordnung der Durchtrittsöffnungen, bei der die Elementarzelle ein flächenzentriertes Quadrat ist (d.h., entsprechend Fig. 3, mit vier identischen Kantenlängen).

Im übrigen kann der wenigstens eine mitzuverwendende Regensiebboden ohne Ablaufsegment wie in der DE-A 10156988 oder wie in der EP-A 1029573 beschrieben gestaltet und in der Kolonne angeordnet sein.

Erfindungsgemäße Verfahren sind demnach solche, in denen Rektifikationskolonnen zum Einsatz kommen, die als trennwirksame Einbauten Böden enthalten, von deren Anzahl mehr als einer (bevorzugt ≥ 10 %, oder ≥ 20 %, oder ≥ 30 %, oder ≥ 40 %, oder ≥ 50 %, oder ≥ 60 %, oder ≥ 75 % aller Böden) und besonders bevorzugt alle erfindungsgemäße Regensiebböden sind, wobei Regensiebböden mit kreisförmigen Durchtrittsöffnungen bevorzugt sind. Günstig ist es, wenn zudem alle Kreisöffnungen innerhalb eines Regensiebbodens den gleichen Querschnitt aufweisen.

Erfindungsgemäß bevorzugt sind Verfahren in Rektifikationskolonnen, die als trennwirksame Einbauten ausschließlich Regensiebböden der erfindungsgemäß zu verwendenden Art enthalten. Dies gilt insbesondere dann, wenn die Durchtrittsöffnungen kreisförmig sind. Vor allem auch dann, wenn sich die Lochdurchmesser gemäß DE-A 10156988 von Boden zu Boden verändern. Sie können aber auch über alle Böden konstant sein. Die Dicke des im erfindungsgemäßen Verfahren zu verwendenden wenigstens einen Regensiebbodens beträgt vorteilhaft 2 mm bis 12 mm.

Das öffnungsverhältnis (Verhältnis der Gesamtfläche aller Durchtrittsöffnungen des wenigstens einen erfindungsgemäß zu verwendenden Regensiebbodens zur Gesamtfläche dieses Regensiebbodens) beträgt erfindungsgemäß zweckmäßig in der Regel 0,1 bis 0,3.

Der Abstand T zweier nächstliegender Schwerpunkte in einem erfindungsgemäßen Regensiebboden beträgt üblicherweise 1,2 d bis 3 d, wobei d die Länge der Längstausdehnung der größeren Durchtrittsöffnung ist (im Fall eines Kreises der Kreisdurchmesser) . d beträgt in typischer Weise 10 bis 80 mm, häufig 10 bis 25 mm.

Enthält die beim erfindungsgemäßen Verfahren eingesetzte Rektifikationskolonne mehrere aufeinanderfolgende erfindungsgemäß mitzuverwendende Regensiebböden, beträgt deren Abstand zweckmäßig 0,1 D bis 0,5 D, wobei D der Durchmesser der Böden bzw. der Innendurchmesser der Rektifikationskolonne ist.

Bevorzugt sind die erfindungsgemäß mitzuverwendenden Regensiebböden aus Edelstahl, insbesondere Edelstahl 1.4571 (nach DIN EN 10020) gefertigt.

Abschließend sei nochmals festgehalten, daß als Ergebnis der vorliegenden Erfindung Regensiebböden mit einer streng regelmäßigen Anordnung der Durchtrittsöffnungen im Bezug auf ihre rektifikative Trennwirkung auf (Meth) acrylmonomere enthaltende Fluide bzw. Flüssigkeiten gegenüber Regensiebböden mit unregelmäßiger Anordnung der Durchtrittsöffnungen überlegen sind. Selbstverständlich können die erfindungsgemäß zu verwendenden Rektifikationskolonnen auch für andere thermische Trennverfahren wie z.B. Extraktionen oder Strippungen oder für rektifikative Auftrennungen anderer Fluide bzw. Flüssigkeiten eingesetzt werden.

Erfindungsgemäß mitzuverwendende Regensiebböden zeichnen sich auch durch eine geringe Neigung zur Polymerisatbildung aus.

Es versteht sich von selbst, daß im Peripheriebereich des wenigstens einen Siebbodens ohne Ablaufsegment nur noch eine Teilabbildung der Elementarzelle auf die dort befindlichen Durchtrittsöffnungen möglich ist.

Das erfindungsgemäße Verfahren eignet sich insbesondere für die in der DE-A 19924532, DE-A 10115277, EP-A 982289, EP-A 982287 und EP-A 982288 beschriebenen Verfahren der fraktionierenden Kondensation bzw. Rektifikation.

Beispiele (die Bodennummerierung steigt innerhalb der Kolonne von unten nach oben)

Beispiel 1

Einer Bodenkolonne, die 40 äquidistante (40 cm) Dual-Flow-Böden aufwies, wurden pro Stunde 120 t eines Gemisches aus 67 Gew.-% Diphyl® (Gemisch aus ca. 25 Gew.-% Diphenyl und ca. 75 Gew.-% Diphenylether), 16 Gew.-% Dimethylphthalat, 15,8 Gew.-% Acrylsäure, 300 gew.-ppm Phenothiazin sowie als Restmenge geringe Mengen an Verbindungen wie Benzaldehyd, Essigsäure, Propionsäure, Furfurale, Ameisensäure und Formaldehyd zugeführt. Die Rektifikationskolonne wurde mit einem Rücklaufverhältnis von 2,2 betrieben. Der Kolonnensumpf wurde mit einem Zwangsumlaufverdampfer beheizt und die Brüden wurden mit einem Einspritzkondensator kondensiert, der auf eine Temperatur von 45$\underline{o}$C gekühlten Rücklauf einspritzte. Die Sumpftemperatur betrug 214°C, der Kopfdruck lag bei 225 mbar. Die Gemischzufuhr erfolgte auf dem 10ten Boden. Auf dem Boden 32 wurde die abgetrennte Acrylsäure entnommen. Die Böden 30, 31 und 32 waren zu Versuchszwecken austauschbar; unterhalb von Boden 30 (auf Boden 29) und unmittelbar oberhalb von Boden 32 war eine Probenentnahmevorrichtung eingebaut, die es erlaubte, eine Probe aus der entsprechenden Flüssigphase zu entnehmen. Oberhalb des 40. Bodens war ein Flüssigkeitsverteiler eingebaut, der pro Stunde ca. 60 t Rücklauf, stabilisiert mit 280 gew.-ppm Phenothiazin, in die einen Durchmesser von 3,50 m aufweisende Rektifikationskolonne verteilte. Der Verteiler bestand aus drei oben geöffneten, parallel angeordneten, quaderförmigen Kästen, die eine Länge von 3 m, eine Breite von 20 cm und eine Höhe von 25 cm aufwiesen. Das obere Ende jedes Kastens war als Zackenwehr ausgeführt.

Nach einer Laufzeit von 10 Tagen wurden an den oben erwähnten Probestellen Proben entnommen. Die entnommenen Acrylsäuren wiesen die in Tabelle 1 aufgeführten Zusammensetzungen auf.

Die Relativanordnung der Durchtrittsöffnungen war in den Regensiebböden 30 bis 32 wie folgt:

- Elementarzelle: in idealer Weise wie in Figur 3, mit $d = a\sqrt{3}$, wobei d die längere und a die kürzere Kante der Elementarzelle ist; d = 58,88 mm.

- die Elementarzelle enthielt fünf Schwerpunkte von Durchtrittsöffnungen, davon vier auf den Eckpunkten der Elementarzelle;

- die Durchtrittsöffnungen waren alle kreisförmig mit einem einheitlichen Zieldurchmesser von 15 mm, der in allen Fällen im Schwankungsbereich $\pm$ 0,01 mm erfüllt war;

- der Abstand zweier nächstliegender Schwerpunkte innerhalb der Elementarzelle betrug 34 mm;

- das Öffnungsverhältnis lag bei 0,16;

- bei regelmäßig wiederkehrender Verschiebung der Elementarzelle entlang ihrer Kanten war in 99,6 % aller Fälle die Position des durch die Verschiebung erzeugten idealen Bildschwerpunktes und die Position des im Siebboden vorhandenen zugehörigen realen Schwerpunktes um $\leq$ 0,1 % der Länge der hälftigen Verschiebungsvektorsumme voneinander entfernt;

- bei regelmäßig wiederkehrender Verschiebung der Elementarzelle entlang ihrer Kanten war in 99,8 % aller Fälle $S_{Rest} \leq 0,1$ %.

Tabelle 1.

|  | unterhalb von Boden 30 | oberhalb von Boden 32 |
| --- | --- | --- |
| Acrylsäure | 99,12 Gew.-% | 99,58 Gew.-% |

Vergleichsbeispiel

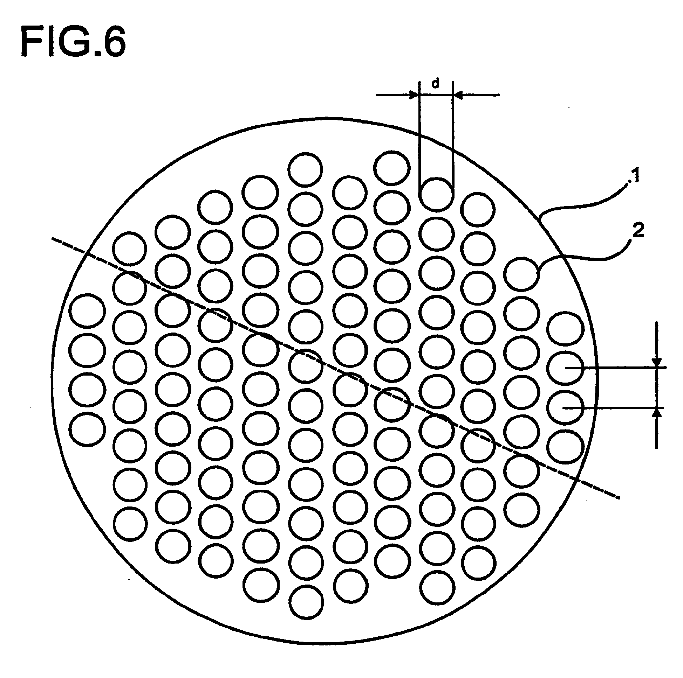

Wie in Beispiel 1, die Böden 30, 31 und 32 wurden jedoch durch Böden gemäß Figur 1 der EP-A 1029573 ersetzt. Figur 6 dieser Schrift, die eine Originalkopie der vorgenannten Figur 1 ist, weist die Abweichungen von der Ideallinie aus.

In allen drei Austausch-Böden waren die Vorgaben der Böden aus Beispiel 1 bis auf die folgenden beiden Ausnahmen erfüllt:

- bei regelmäßig wiederkehrender Verschiebung der Elementarzelle entlang ihrer Kanten war nur in 89 % aller Fälle die Position des durch die Verschiebung erzeugten idealen Bildschwerpunktes und die Position des im Siebboden vorhandenen zugehörigen realen Schwerpunktes um $\leq$ 1 % der Länge der hälftigen Verschiebungsvektorsuamme voneinander entfernt;

- bei regelmäßig wiederkehrender Verschiebung der Elementarzelle entlang ihrer Kanten war in nur 86 % aller Fälle $S_{Rest} \leq 1$ %.

Nach einer Laufzeit von 10 Tagen wurden wie in Beispiel 1 Proben entnommen. Die Analysenergebnisse lauten:

Tabelle 2

|  | unterhalb von Boden 30 | oberhalb von Boden 32 |
| --- | --- | --- |
| Acrylsäure | 99,13 Gew.-% | 99,39 Gew.-% |

Beispiel 2

Wie in Beispiel 1. Alles war identisch, die Elementarzelle der Böden 30 bis 32 war jedoch quadratisch flächenzentriert (wie in Fig. 3, aber mit a = d = 45 mm).

Nach einer Laufzeit von 10 Tagen wurden wie in Beispiel 1 Proben entnommen. Die Analysenergebnisse lauten:

Tabelle 3

|  | unterhalb von Boden 30 | oberhalb von Boden 32 |
| --- | --- | --- |
| Acrylsäure | 99,09 Gew.-% | 99,59 Gew.-% |

Beispiel 3

Wie in Beispiel 1. Alles war identisch, die Elementarzelle der Böden 30 bis 32 war jedoch rechteckig wie in Figur 1. Die Kantenlängen betrugen 28 mm und 35 mm.

Nach einer Laufzeit von 10 Tagen wurden wie in Beispiel 1 Proben entnommen.

Die Analysenergebnisse lauten:

Tabelle 4

|  | unterhalb von Boden 30 | oberhalb von Boden 32 |
|---|---|---|
| Acrylsäure | 99,15 Gew.-% | 99,52 Gew.-% |

Entsprechend den durchgeführten Versuchen ergibt sich folgende Abstufung der Wirksamkeit:

Beispiel 2 > Beispiel 1 > Beispiel 3 > Vergleichsbeispiel.

**Patentansprüche**

1. Verfahren der rektifikativen Auftrennung von (Meth)acrylmonomere enthaltenden Fluiden in einer Rektifikationskolonne, die als trennwirksame Einbauten Böden enthält,
**dadurch gekennzeichnet,**
**dass** von der Anzahl der Böden mehr als einer ein Siebboden ohne Ablaufsegment ist, dessen Anordnung der Schwerpunkte der im Siebboden enthaltenen Durchtrittsöffnungen als auch die Anordnung der Durchtrittsöffnungen selbst eine regelmäßige ist, die sich aus einer Grundmenge dieser Schwerpunkte und Durchtrittsöffnungen **dadurch** erzeugen läßt, dass man im Siebboden eine viereckige Elementarzelle definiert, die diese Grundmenge enthält und von deren vier Kanten jeweils zwei zueinander parallel und gleich lang sind, und diese Elementarzelle regelmäßig wiederkehrend entlang ihrer Kanten verschiebt, wobei die Länge des Verschiebungsvektors jeweils die Länge der Kante der Elementarzelle ist, entlang der die Verschiebung erfolgt, mit der Maßgabe,
**dass** einerseits bei der regelmäßig wiederkehrenden Verschiebung der Elementarzelle entlang ihrer Kanten in wenigstens 90 % aller Fälle die Position des im Siebboden vorhandenen zugehörigen idealen Bildschwerpunktes und die Position des im Siebboden vorhandenen zugehörigen realen Schwerpunktes um ≤ 1 % der Hälfte der Längen-Summe der beiden möglichen Verschiebungsvektoren voneinander entfernt sind,
und andererseits bei der regelmäßig wiederkehrenden Verschiebung der Elementarzelle entlang ihrer Kanten in wenigstens 90 % aller Fälle die Fläche der durch die Verschiebung erzeugten idealen Durchtrittsöffnung und die Fläche der im Siebboden vorhandenen zugehörigen realen Durchtrittsöffnung so weitgehend miteinander überlappen, dass die Summe $S_{Rest}$, gebildet aus der nicht überlappenden Restfläche der durch die Verschiebung erzeugten idealen Durchtrittsöffnung und der nicht überlappenden Restfläche der zugehörigen realen Durchtrittsöffnung, bezogen auf die Fläche der durch die Verschiebung erzeugten idealen Durchtrittsöffnung, ≤ 1 % beträgt.

2. Verfahren nach Anspruch 1, **dadurch gekennzeichnet, dass** die Durchtrittsöffnungen kreisförmig sind.

3. Verfahren nach Anspruch 1, **dadurch gekennzeichnet, dass** die Rektifikationskolonne als trennwirksame Einbauten nur Siebböden ohne Ablaufsegment gemäß Anspruch 1 enthält.

4. Verfahren nach Anspruch 3, **dadurch gekennzeichnet, dass** die Durchtrittsöffnungen in allen Siebböden kreisförmig sind.

5. Verfahren nach einem der Ansprüche 1 bis 4, **dadurch gekennzeichnet, dass** das Fluid als (Meth)acrylmonomere Acrylsäure enthält.

6. Verfahren nach Anspruch 5, **dadurch gekennzeichnet, dass** der Acrylsäuregehalt des Fluids 2 bis 5 Gew.-%, oder 10 bis 35 Gew.-%, oder ≥ 95 Gew.-% beträgt.

7. Verfahren nach einem der Ansprüche 1 bis 6, **dadurch gekennzeichnet, dass** das (Meth)acrylmonomere enthaltende Fluid Diphenyl als Lösungsmittel enthält.

8. Verfahren nach einem der Ansprüche 1 bis 7, **dadurch gekennzeichnet, dass** die Elementarzelle ein flächenzentriertes Quadrat ist.

9. Verfahren nach einem der Ansprüche 1 bis 7, **dadurch gekennzeichnet, dass** die Elementarzelle ein Rechteck ist.

10. Verfahren nach einem der Ansprüche 1 bis 7, **dadurch gekennzeichnet, dass** die Elementarzelle eine Raute ist.

11. Verfahren nach einem der Ansprüche 1 bis 10, **dadurch gekennzeichnet, dass** das Fluid eine Flüssigkeit ist.

12. Verfahren nach einem der Ansprüche 1 bis 11, **dadurch gekennzeichnet, dass** das Fluid ein Gasgemisch ist.

**Claims**

1. A process for rectificatively separating (meth)acrylic monomer-containing fluids in a rectification column which comprises trays as separating intervals,
   wherein
   more than one of the number of the trays is a sieve tray without a runoff segment, wherein both the arrangement of the centers of the passages present in the sieve tray and the arrangement of the passages themselves are regular and can be obtained from a basic number of these centers and passages by defining a rectangular unit cell in the sieve tray which contains this basic number and has four edges, of which two in each case are parallel to one another and of equal length, and this unit cell shifts regularly and repeatedly along its edges, the length of the shifting vector in each case being the length of the edge of the unit cell along which the shifting is effected, with the proviso that firstly, in the regularly repeating shift of the unit cell along its edges, in at least 90% of all cases, the position of the corresponding ideal image center present in the sieve tray and the position of the corresponding real center present in the sieve tray are removed by $\leq$ 1% of half of the total length of the two possible shift vectors, and secondly, in the regularly repeating shift of the unit cell along its edges, in at least 90% of all cases, the area of the ideal passage orifice formed by the shift and the area of the corresponding real passage orifice present in the sieve tray overlap with one another to such a substantial extent that the sum $S_{residual}$, formed from the non-overlapping residual area of the ideal passage orifice formed by the shift and the non-overlapping residual area of the corresponding real passage orifice, based on the area of the ideal passage orifice formed by the shift, is $\leq$ 1%.

2. The process according to claim 1, wherein the passages are circular.

3. The process according to claim 1, wherein the only separating internals of the rectification column are sieve trays without runoff segment according to claim 1.

4. The process according to claim 3, wherein the passages in all sieve trays are circular.

5. The process according to any of claims 1 to 4, wherein the (meth)acrylic monomer contained in the fluid is acrylic acid.

6. The process according to claim 5, wherein the acrylic acid content of the fluid is from 2 to 5% by weight, or from 10 to 35% by weight, or $\geq$ 95% by weight.

7. The process according to any of claims 1 to 6, wherein the (meth)acrylic monomer-containing fluid comprises diphenyl as solvent.

8. The process according to any of claims 1 to 7, wherein the unit cell is a face-centered square.

9. The process according to any of claims 1 to 7, wherein the unit cell is a rectangle.

10. The process according to any of claims 1 to 7, wherein the unit cell is a rhombus.

11. The process according to any of claims 1 to 10, wherein the fluid is a liquid.

12. The process according to any of claims 1 to 11, wherein the fluid is a gas mixture.

**EP 1 521 623 B1**

**Revendications**

1. Procédé de séparation rectificative de fluides contenant des monomères (méth)acryliques dans une colonne de rectification, qui contient des plateaux en tant que composants de séparation,

   **caractérisé en ce que**

   sur le nombre des plateaux, plusieurs sont des plateaux perforés sans segment d'évacuation, dont l'agencement des centres de gravité des ouvertures de passage contenues dans le plateau perforé, ainsi que l'agencement des ouvertures de passage elles-mêmes, est un agencement régulier qui peut être généré à partir d'un ensemble de référence de ces centres de gravité et de ces ouvertures de passage en définissant une cellule élémentaire quadrangulaire dans le plateau perforé, qui contient cet ensemble de référence et dont les quatre arêtes sont deux à deux parallèles et de même longueur, et en déplaçant répétitivement cette cellule élémentaire de manière régulière le long de ses arêtes, la longueur du vecteur de déplacement étant à chaque fois la longueur de l'arête de la cellule élémentaire le long de laquelle le déplacement a lieu, à condition

   d'une part que, lors du déplacement répétitif régulier de la cellule élémentaire le long de ses arêtes, dans au moins 90 % de tous les cas, la position du centre de gravité idéal et la position du centre de gravité réel correspondant présent dans le plateau perforé soient écartées l'une de l'autre de ≤ 1 % de la moitié de la somme des longueurs des deux vecteurs de déplacement possibles,

   et, d'autre part, que, lors du déplacement répétitif régulier de la cellule élémentaire le long de ses arêtes, dans au moins 90 % de tous les cas, la surface de l'ouverture de passage idéale générée par le déplacement et la surface de l'ouverture de passage réelle correspondante présente dans le plateau perforé se chevauchent dans une mesure telle que la somme $S_{rés}$, formée par la surface résiduelle non chevauchée de l'ouverture de passage idéale générée par le déplacement et la surface résiduelle non chevauchée de l'ouverture de passage réelle correspondante, par rapport à la surface de l'ouverture de passage idéale générée par le déplacement, soit ≤ 1 %.

2. Procédé selon la revendication 1, **caractérisé en ce que** les ouvertures de passage sont circulaires.

3. Procédé selon la revendication 1, **caractérisé en ce que** la colonne de rectification ne contient en tant que composants de séparation que des plateaux perforés sans segment d'évacuation selon la revendication 1.

4. Procédé selon la revendication 3, **caractérisé en ce que** les ouvertures de passage sont circulaires dans tous les plateaux perforés.

5. Procédé selon l'une quelconque des revendications 1 à 4, **caractérisé en ce que** le fluide contient de l'acide acrylique en tant que monomères (méth)acryliques.

6. Procédé selon la revendication 5, **caractérisé en ce que** la teneur en acide acrylique du fluide est de 2 à 5 % en poids, ou de 10 à 35 % en poids, ou ≥ 95 % en poids.

7. Procédé selon l'une quelconque des revendications 1 à 6, **caractérisé en ce que** le fluide contenant des monomères (méth)acryliques contient du diphényle en tant que solvant.

8. Procédé selon l'une quelconque des revendications 1 à 7, **caractérisé en ce que** la cellule élémentaire est un carré à faces centrées.

9. Procédé selon l'une quelconque des revendications 1 à 7, **caractérisé en ce que** la cellule élémentaire est un rectangle.

10. Procédé selon l'une quelconque des revendications 1 à 7, **caractérisé en ce que** la cellule élémentaire est un losange.

11. Procédé selon l'une quelconque des revendications 1 à 10, **caractérisé en ce que** le fluide est un liquide.

12. Procédé selon l'une quelconque des revendications 1 à 11, **caractérisé en ce que** le fluide est un mélange gazeux.

# FIG.1

# FIG.2

# FIG.3

# FIG.4

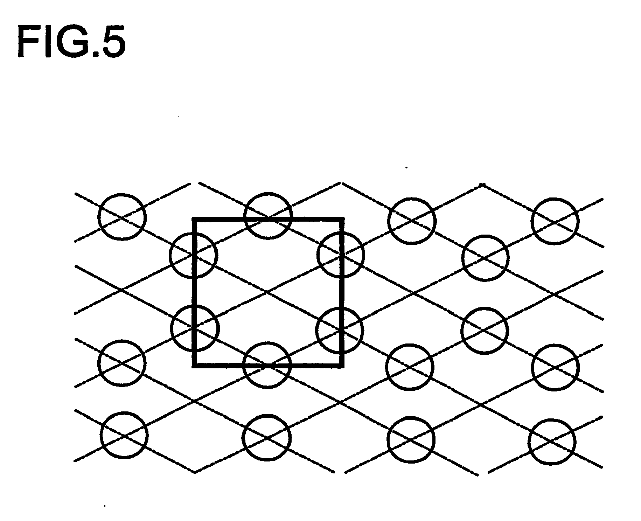

# FIG.5

# FIG.6

## IN DER BESCHREIBUNG AUFGEFÜHRTE DOKUMENTE

**In der Beschreibung aufgeführte Patentdokumente**

- EP 717019 A
- EP 1125912 A
- EP 982289 A
- EP 982287 A
- DE 19606877 A
- DE 1011527 A
- DE 10224341 A
- DE 10218419 A
- DE 19924532 A
- DE 10156988 A
- EP 1029573 A
- DE 10159823 A
- DE 10115277 A
- EP 982288 A